# EUROPEAN PATENT APPLICATION

(11) **EP 3 421 996 A1**
(43) Date of publication of application: **02.01.2019**
(21) Application number: 17756557.9
(22) Date of filing: 22.02.2017
(51) Int. Cl.: G01N 33/53, C07K 16/36, C12Q 1/56

(54) **MEASUREMENT REAGENT FOR CROSSLINKED FIBRIN DEGRADATION PRODUCT, AND MEASUREMENT METHOD**

(30) Priority: 22.02.2016 JP 2016031407
(71) Applicant: LSI Medience Corporation, Tokyo 101-8517 (JP)
(72) Inventor: NAGAHAMA Yutaka, Tokyo 101-8517 (JP); NOZAKI Junko, Tokyo 101-8517 (JP); SAKURAI George, Tokyo 101-8517 (JP)
(74) Representative: Cohausz & Florack
(86) International application number: PCT/JP2017/006677
(87) International publication number: WO 2017/146119

(57) **Abstract**

Provided are a reagent and a method that allows a more accurate measurement of plasmin degradation products of cross-linked fibrin (XDP). The measurement reagent of the present invention comprises (1) an anti-XDP antibody that reacts with XDP, but does not react with fibrinogen, and fragment X, fragment Y, fragment D₁, and fragment E₃, which are plasmin degradation products of fibrinogen, and (2) a calcium chelating agent.

## Description

### TECHNICAL FIELD

The present invention relates to a measurement reagent for plasmin degradation products of cross-linked fibrin, and a measurement method for it.

### BACKGROUND ART

The fact that cross-linked fibrin degradation products (XDP) are detected in blood means that a thrombus has dissolved. The measurement of XDP is widely used for diagnosis of disseminated intravascular coagulation syndromes (DIC), in which systemic activation of coagulation and fibrinolytic activation are observed. On the other hand, it is also used for exclusion diagnosis of deep vein thrombosis (DVT) with local intravascular thrombus formation and limited fibrinolytic activation, therefor the measurement range required for the XDP measurement system tends to be widened from a low concentration to a high concentration.

An antibody disclosed in Patent literature 1 is known as an antibody recognizing XDP. Since this antibody does not react with fragments other than XDP (D dimer), accurate measurement of XDP is expected. From the reactivity and the like of the antibody, it is considered that the recognition site of the antibody is present in the D domain and/or the E domain of XDP having E-D bonds, so the E-D bond is important for accurate measurement of XDP.

### CITATION LIST

### PATENT LITERATURE

[Patent literature 1] WO 2011/125875

### SUMMARY OF INVENTION

### TECHNICAL PROBLEM

On the other hand, the inventors found that, when XDP was measured using the antibody having the properties shown in Patent literature 1, there was a possibility that a fibrin polymer and soluble fibrin (SF), which were contained in a sample and did not have a cross-linked structure on the D domain, were recognized and measured, as a problem specific to antibodies specifically recognizing the E-D bond.

Since these substances such as a fibrin polymer and SF have partially structural similarities to XDP, but are quite different in origin and properties, a false positive due to the recognition of a fibrin polymer and SF can be a big problem. Therefore, it is necessary to distinguish cross-linked fibrin degradation product (XDP) from a fibrin polymer and SF, and to measure.

### SOLUTION TO PROBLEM

The inventors conducted intensive studies, and as a result, clarified the properties of the antibody, and made it possible to carry out the measurement capable of distinguishing them. More particularly, the inventors found that the recognition site of the antibody was present in the E domain, and that the calcium concentration was important for a useful XDP measurement using the antibody.

Calcium is an ion that plays an important role in blood coagulation, and it has been reported that there are two calcium binding sites in the D domain and one calcium binding site in the E domain. As a result of examination by the inventors, it is considered that since calcium on the fibrin polymer chelated and is not present, the structure of the fibrin polymer changes, and the reactivity also changes.

The present invention relates to:
[1] a measurement reagent for plasmin degradation products of cross-linked fibrin (XDP), said measurement reagent comprising:
   (1) an anti-XDP antibody that reacts with XDP, but does not react with fibrinogen, and fragment X, fragment Y, fragment D₁, and fragment E₃, which are plasmin degradation products of fibrinogen, and
   (2) a calcium chelating agent; and
[2] a measurement method for plasmin degradation products of cross-linked fibrin (XDP), comprising carrying out a reaction of XDP with an anti-XDP antibody in the presence of a calcium chelating agent.

### ADVANTAGEOUS EFFECTS OF INVENTION

The present invention enables a more accurate measurement of XDP.

### BRIEF DESCRIPTION OF THE DRAWINGS

[Fig. 1-A] Fig. 1-A is a diagram schematically showing the structures of a) fibrinogen, b) desAA fibrin monomer, and c) fibrin polymer.
[Fig. 1-B] Fig. 1-B is a diagram schematically showing the structure of d) cross-linked fibrin.
[Fig. 1-C] Fig. 1-C is a diagram schematically showing the structures of e) cross-linked fibrin degradation products
   (DD/E), f) isolated DD after dissociation of DD/E, g) isolated fragments E₁ and E₂ after dissociation of DD/E, h) fragment E₃, i) fragment D₁, j) fragment D₃, k) D₁-E₁-D₁, and 1)D₁-E₁.
[Fig. 2-A] Fig. 2-A is a chromatogram showing the results of fractionation of a mixture of human D₁ and human E₁₊₂ on a Superdex200 HR column.
[Fig. 2-B] Fig. 2-B is a chromatogram showing the results of fractionation of a mixture of human D₁ and human E₃ on a Superdex200 HR column.
[Fig. 3-A] Fig. 3-A is a chromatogram showing the results of fractionation of a mixture of bovine D₁ (bD1) and human E₁₊₂ (hE₁₊₂) on a Superdex200 HR column.
[Fig. 3-B] Fig. 3-B is a chromatogram showing the results of fractionation of a mixture of ovine D₁ (oD1) and human E₁₊₂ (hE₁₊₂) on a Superdex200 HR column.
[Fig. 4] Fig. 4 is a graph showing the results obtained by reacting human DD/E or human D-E-D complex with an immobilized MIF-220 antibody and detecting them by ELISA, in order to examine the influence of a Ca²⁺ ion or EDTA.

### DESCRIPTION OF EMBODIMENTS

The measurement reagent and the measurement method of the present invention are characterized in that (1) an anti-XDP antibody that reacts with plasmin degradation products of cross-linked fibrin (XDP), but does not react with fibrinogen, and fragment X, fragment Y, fragment D₁, and fragment E₃, which are plasmin degradation products of fibrinogen, and (2) a calcium chelating agent are used, and the reaction of XDP with the anti-XDP antibody is carried out in the presence of the calcium chelating agent.

The term "plasmin degradation products of cross-linked fibrin (cross-linked fibrin is also called "stabilized fibrin")" as used herein is also called "D dimer", "D-D dimer", or "DD/E complex", and means a DD/E monomer as the smallest unit, and its polymer (DD/E polymer, for example, DXD/YY, YXY/DXXD, and DXXY/YXXD).

### <<Anti-XDP antibody>>

The anti-XDP antibody used in the present invention:
(1) reacts with XDP,
(2) does not react with fibrinogen, and
(3) does not react with any of the plasmin degradation products of fibrinogen, i.e., fragment X, fragment Y, fragment D₁, and fragment E₃, and preferably,
(4) does not react with any of the fragments obtained by dissociating the DD/E monomer, i.e., fragment DD, fragment E₁, and fragment E₂.

As the anti-XDP antibody used in the present invention, an antibody that recognizes a three-dimensional structure newly generated on the E domain side by the assembly of the E domain and the D domain may be exemplified.

The anti-XDP antibody used in the present invention may be obtained by a known method. It may be a monoclonal antibody (MoAb) or a polyclonal antibody (PoAb), so long as it has the desired reactivity, but the monoclonal antibody is preferable. As the antibody, the antibody per se may be used, or an antibody fragment comprising the antigen-binding site, for example, Fab, Fab', F(ab')₂, Fv, or the like, may be used. These antibody fragments may be obtained by digesting the antibody with a protease in accordance with a conventional method, and in accordance with conventional methods of protein separation and purification.

As an antigen for preparing the anti-XDP antibody used in the present invention, for example, the DD/E monomer or the DD/E polymer may be used. As the DD/E polymer, a dimer, a trimer, a tetramer, or a pentamer may be exemplified. When it is more than a hexamer, it becomes less soluble in water. The antigen may be prepared from fibrinogen in accordance with a known method. Alternatively, the antigen may be obtained by purification from a human or the like, or by genetic engineering technique. Furthermore, a commercially available product may be used.

The anti-XDP antibody used in the present invention may be obtained by confirming the reactivities against XDP; fibrinogen; plasmin degradation products of fibrinogen, i.e., fragment X, fragment Y, fragment D₁, and fragment E₃; and fragment DD, fragment E₁, and fragment E₂ obtained by dissociating the DD/E monomer, by a known immunological analysis method as shown in Examples described below.

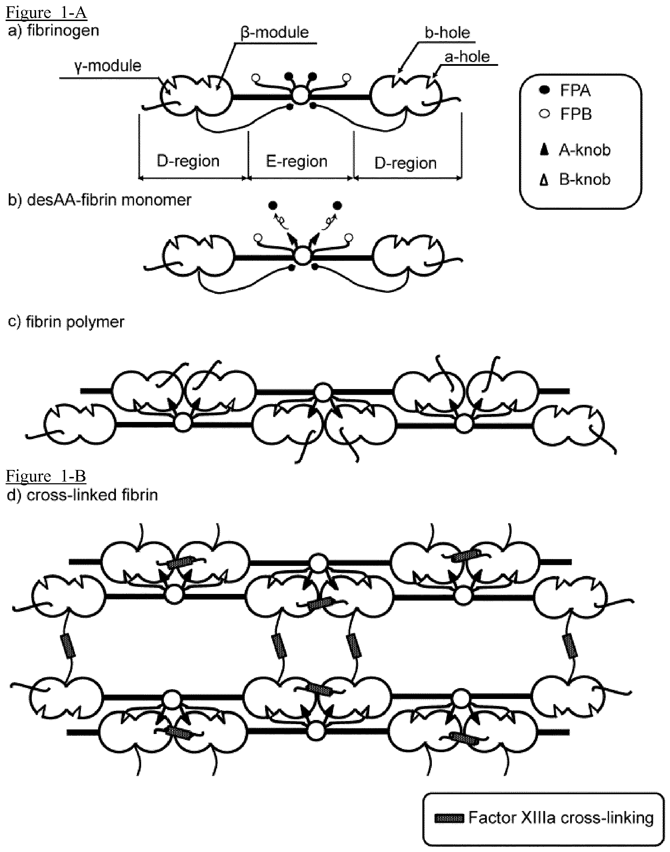

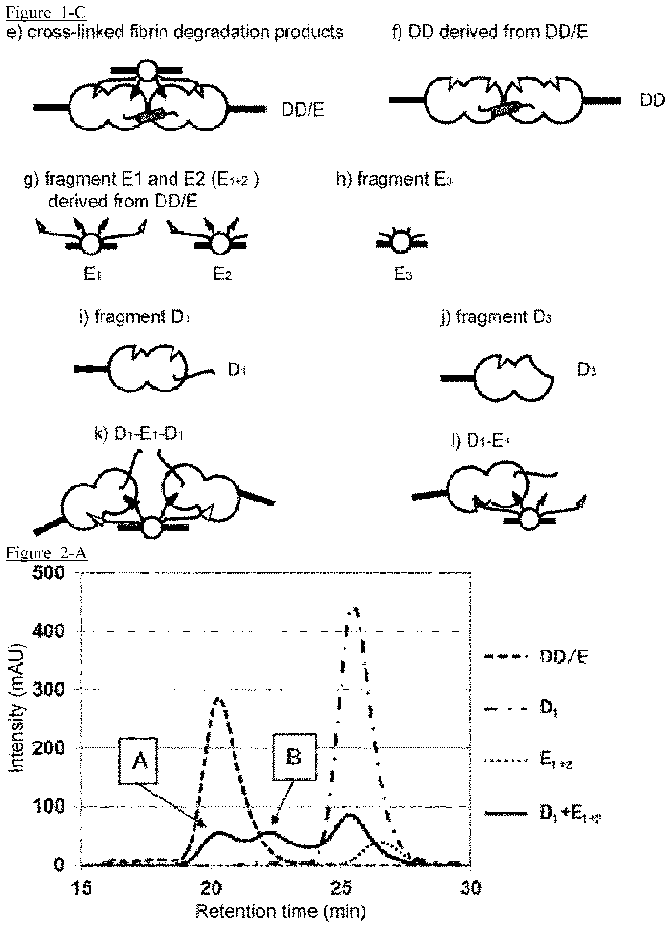

In the anti-XDP antibody used in the present invention, with respect to the reactivity to a reconstructed D-E-D complex [D₁-E-D₁: see k) of Figure 1-C] obtained by mixing fragment E₁ and/or fragment E₂ (i.e., either fragment E₁ or fragment E₂, or a mixture of fragment E₁ and fragment E₂) with fragment D₁ and the reactivity to the DD/E monomer, it is preferable to confirm the influence of Ca²⁺ ion concentration. More particularly, it is preferable to confirm that the reactivity to the DD/E monomer is not significantly affected by Ca²⁺ ion concentration, and that the reactivity to the reconstructed D-E-D complex is significantly affected by Ca²⁺ ion concentration, and is significantly reduced when the Ca²⁺ ion concentration is lower than a certain value (or in the presence of a calcium chelating agent, such as EDTA).

The major structural difference between the DD/E monomer and the reconstructed D-E-D complex is that the DD/E monomer has cross-linking between the D-D. The D-E-D complex does not have cross-linking between the D-D, and may be regarded as the smallest unit that characterizes, for example, fibrin polymer degradation products. Therefore, in the anti-XDP antibody, wherein the reactivity to the D-E-D complex is significantly affected by Ca²⁺ ion concentration, even if fibrin polymer degradation products not having cross-linking between the D-D is present in a sample, the reactivity to the fibrin polymer degradation products can be significantly reduced in the presence of a calcium chelating agent, while the reactivity to the DD/E monomer is maintained, and as a result, it enables a more specific measurement of XDP.

### <<Measurement method and reagent>>

In the measurement method of the present invention, a known immunological measurement method may be used, except that the aforementioned anti-XDP antibody is used, and the reaction of XDP with the anti-XDP antibody is carried out in the presence of a calcium chelating agent. For example, the measurement method may comprise the step of preparing a sample, which may contain XDP, and the anti-XDP antibody; the step of bringing the sample into contact with the anti-XDP antibody in the presence of a calcium chelating agent to form an immunological complex; and the step of analyzing the immunological complex or a signal derived from the immunological complex. More particularly, a turbidimetric immunoassay (TIA), an enzyme immunoassay (EIA), a radioimmunoassay (RIA), a latex agglutination method, a fluorescent immunoassay, an immunochromatography, and the like, may be exemplified.

It is common to use sodium citrate as an anticoagulant in the examination of coagulation items. The Japanese Association of Clinical Laboratory Physicians also recommends the use of sodium citrate as an anticoagulant in coagulation tests, and it is well-known that EDTA salts are not suitable for coagulation tests. This is because, in tests such as activated partial thromboplastin time (APTT), prothrombin time (PT), or the like, the results are longer than citric acid, and it is considered that it has a serious effect on test results, and is unsuitable for accurate quantification.

The calcium chelating agent is a substance that forms coordinate bonds with a metal ion so as to sandwich the metal ion at the center, and a compound capable of coordinating calcium may be used in the present invention.

As the calcium chelating agent which may be used in the present invention, for example, aminopolycarboxylic acid type chelating agents, aromatic or aliphatic carboxylic acid type chelating agents, amino acid type chelating agents, ether carboxylic acid type chelating agents, phosphonic acid type chelating agents, hydroxycarboxylic acid type chelating agents, polymer electrolyte (including oligomer electrolyte) type chelating agents, polyalcohols, nitrogen-containing chelating agents such as dimethylglyoxime or the like, sulfur-containing chelating agents such as thioglycolic acid or the like, or the like, may be exemplified.

The form of these chelating agents is arbitrary. In the case of an acidic chelating agent, it may be in the form of a free acid, or in the form of a salt, such as a sodium salt, a potassium salt, an ammonium salt, or the like. Furthermore, it may be in the form of a hydrolyzable ester derivative thereof.

As the aminopolycarboxylic acid type chelating agents, for example, ethylenediamine tetraacetic acid (EDTA), ethylenediamine diacetic acid, hydroxyethylethylenediamine triacetic acid (HEDTA), dihydroxyethylethylenediaminetetraacetic acid (DHEDDA), nitrilotriacetic acid (NTA), hydroxyethyliminodiacetic acid (HIDA), β-alaninediacetic acid, cyclohexane diamine tetraacetic acid, nitrilotriacetic acid, iminodiacetic acid, N-(2-hydroxyethyl) iminodiacetic acid, diethylenetriaminepentaacetic acid, N- (2-hydroxyethyl)ethylenediaminetriacetic acid, glycol ether diamine tetraacetic acid, glutamic acid diacetic acid, aspartic acid diacetic acid, methylglycine diacetic acid, iminodisuccinic acid, serine diacetic acid, hydroxyiminodisuccinic acid, dihydroxyethylglycine, aspartic acid, and glutamic acid; and their salts, and their derivatives such as esters or the like, may be exemplified.

As the aromatic or aliphatic carboxylic acid type chelating agents, for example, glyoxylic acid, oxalic acid, malonic acid, succinic acid, glutaric acid, adipic acid, pimelic acid, sebacic acid, azelaic acid, itaconic acid, aconitic acid, pyruvic acid, gluconic acid, pyromellitic acid, benzopolycarboxylic acid, cyclopentanetetracarboxylic acid, salicylic acid, acetylsalicylic acid, hydroxybenzoic acid, aminobenzoic acid (including anthranilic acid), phthalic acid, fumaric acid, trimellitic acid, gallic acid, and hexahydrophthalic acid; and their salts and their derivatives, may be exemplified.

As the amino acid type chelating agents, for examples, glycine, serine, alanine, lysine, cystine, cysteine, ethionine, tyrosine, and methionine; and their salts and their derivatives, may be exemplified.

As the ether carboxylic acid type chelating agents, ether carboxylic acid salts such as carboxymethyl tartronic acid (CMT) and carboxymethyl oxysuccinic acid (CMOS), carboxymethyl tartronate, carboxymethyl oxysuccinate, oxydisuccinate, tartaric acid monosuccinate, and disuccinate tartrate; and their salts and derivatives, may be exemplified.

As the phosphonic acid type chelating agents, for example, iminodimethylphosphonic acid, alkyldiphosphonic acid, 1-hydroxyethane-1,1-diphosphonic acid, and phytic acid; and their salts and derivatives, may be exemplified.

As the hydroxycarboxylic acid type chelating agents, for example, malic acid, citric acid, glycolic acid, gluconic acid, heptonic acid, tartaric acid, and lactic acid; and their salts and derivatives, may be exemplified.

As the polymer electrolyte (including oligomer electrolyte) type chelating agents, for example, an acrylic acid polymer, a maleic anhydride polymer, an α-hydroxyacrylic acid polymer, and an itaconic acid polymer; a copolymer composed of two or more constituent monomers of these polymers; and an epoxy succinic acid polymer, may be exemplified.

As the polyalcohols, for example, ethylene glycol, pyrocatechol, pyrogallol, bisphenol, and tannic acid; and their derivatives, may be exemplified.

In particular, ethylenediamine tetraacetic acid (EDTA), oxalic acid, citric acid, tartaric acid, gluconic acid, nitrilotriacetic acid (NTA), ethylenediamine, diethylenetriamino pentaacetic acid (DTPA), dihydroxyethylene glycine, triethanolamine, hydroxyethylenediaminetetraacetic acid, carboxymethyltartronic acid (CMT), carboxymethyloxysuccinic acid (CMOS), BAPTA, Bicine, CyDTA, GEDTA (EGTA), HIDA, IDA, NTPO, TTHA, TPEN, bipyridine, phenanthroline, porphyrin, crown ether, and the like, are preferable.

These chelating agents may be used alone, or as a combination of two or more compounds.

The content of the chelating agent may be appropriately set in consideration of its chelating ability. In the measurement method of the present invention, the concentration of the calcium chelating agent in the reaction system between XDP and the anti-XDP antibody is preferably 0.01 to 30 mmol/L, more preferably 0.2 to 30 mmol/L, and still more preferably 0.2 to 2 mmol/L, at the time of the antigen-antibody reaction, in the case of EDTA, as an example. The lower limit of the EDTA concentration is not limited, so long as it is 0.001 mmol/L or more. In connection with this, it is preferably 0.002 mmol/L or more, more preferably 0.005 mmol/L or more, still more preferably 0.01 mmol/L or more, still more preferably 0.02 mmol/L or more, 0.05 mmol/L or more, and most preferably 0.02 mmol/L or more. The EDTA concentration does not have an upper limit, and it may be appropriately set for those skilled in the art, but it is preferable to set the EDTA concentration within the range not affecting the reaction of the anti-XDP antibody. Each lower limit and each upper limit above can be arbitrarily combined as desired.

Calcium is an ion that plays an important role in blood coagulation, and it has been reported that there are two calcium binding sites in the D domain and one calcium binding site in the E domain. As a result of examination by the inventors, it is considered that since calcium on the fibrin polymer chelated and is not present, the structure of the fibrin polymer changes, and the reactivity also changes.

The measurement reagent of the present invention may be configured similarly to known immunological measurement reagents, except that it comprises the anti-XDP antibody and the calcium chelating agent. In addition to the anti-XDP antibody and the calcium chelating agent, for example, a buffer, a sensitizer, a surfactant, an inorganic salt, and the like, may be appropriately added.

The reagent form may be a two-reagent system consisting of a first reagent and a second reagent, or a reagent form consisting of one reagent. The two-reagent system consisting of a first reagent and a second reagent is preferable from the viewpoint of measurement accuracy and the like, and a measurement reagent based on a latex agglutination method will be exemplified below.

The first reagent is composed of a buffer, and the second reagent is composed of antibody-sensitized latex particles, and a sensitizer, a surfactant, an inorganic salt, and the like, may be appropriately added. As a preferable example, a reagent consisting of the first reagent consisting of a buffer, a sensitizer, and an inorganic salt, and the second reagent consisting of antibody-sensitized latex particles may be exemplified. The calcium chelating agent may be added to either the first reagent or the second reagent, or both the first reagent and the second reagent, but it is preferable to add the calcium chelating agent to the first reagent.

The concentration of the calcium chelating agent in the reagent varies depending on, for example, the reagent constitution, the order of addition, the volume added, and the like, but it may be appropriately determined so as to be within the above concentration range in the reaction system between XDP and the anti-XDP antibody.

### EXAMPLES

The present invention will now be further illustrated by, but is by no means limited to, the following Examples.

### <<Example 1: Preparation of fibrin/fibrinogen degradation products>>

Cross-linked fibrin degradation products (XDP) have a structure of (DD/E)n, wherein n DD/Es are linearly connected, wherein the DD/E is the smallest unit and n is an integer. In the Examples below, XDPs (n≥2) are represented as polymeric XDP, and XDP (n=1) is represented as DD/E. The polymeric XDP and DD/E were prepared, mainly in accordance with Stephanie A. Olexa and Andrei Z. Budzynski (1978), Circulation, Suppl. 58, 119, and Olexa et al. (1979), Biochim. Biophys. Acta 576, 39-50. Bovine thrombin (Mochida Pharmaceutical Co., Ltd.) and calcium chloride were added to human fibrinogen (Enzyme Research Laboratories), and the reaction was carried out at 37°C for 2 hours to convert fibrinogen into fibrin. This was centrifuged to separate fibrin from noncoagulable substances. Fibrin was floated in a Tris-HCl buffer (pH7.8) containing calcium chloride. Human plasmin (Chromogenix) was added to the suspension at 37°C. The degradation reaction was stopped by adding aprotinin to the suspension, and the suspension was passed through a lysine sepharose column to remove plasmin from the filtrate. The filtrate was a mixture of polymeric XDP and DD/E, of which the molecular weights are different from each other. The filtrate was subjected to a Sephacryl S-300 (S-300) column equilibrated with a Tris-HCl buffer (pH 7.5) containing calcium chloride (solution A), and was fractionated by molecular sieve chromatography developed with solution A. The obtained fractions were subjected to electrophoresis by SDS-PAGE, and Western blotting to identify and separate the polymeric XDP fraction and the DD/E fraction.

With respect to human fibrinogen degradation products (X, Y, D₁, and E₃), after calcium chloride was added to human fibrinogen (Enzyme Research Laboratories), human plasmin (Chromogenix) was further added, and the reaction was carried out at 37°C for 2 hours. The reaction was stopped by adding aprotinin (Pentapharm.). The product after stopping the reaction was subjected to a Sephacryl S-300 (S-300) column equilibrated with a Tris-HCl buffer (pH 7.5), and was fractionated into fragment X, fragment Y, fragment D₁, and fragment E₃ by gel filtration.

With respect to the preparation of a DD fraction (DD) and an E₁₊₂ fraction (a mixture of E₁ and E₂: E₁₊₂), the previously obtained DD/E was allowed to stand at 37°C in a 3 mol/L urea-50 mmol/L citric acid (pH 5.5) solution for 4 hours. This was subjected to a Sepharose CL-6B column equilibrated with a 50 mmol/L Tris-HCl buffer (pH 7.4)-28 mmol/L sodium citrate-0.1 mol/L sodium chloride solution, and was developed with the solution. The obtained fractions were subjected to electrophoresis by SDS-PAGE, and Western blotting to identify and separate fragment DD, and fragment E₁ and fragment E₂. Bovine and ovine DD/E and D₁ were also prepared in the same manner. Human D₃ was prepared in accordance with Varadi A. and Scheraga H.A., Biochemistry 25: 519-28, 1986. Schematic diagrams of the fractions are shown in Figures 1-A to 1-C.

The fractions were basically suspended in a 50 mmol/L Tris buffer (pH 8.0) containing 0.9% NaCl (hereinafter referred to as TBS). The purity of each degradation product was confirmed by Western blotting and HPLC under reducing/non-reducing conditions, and the protein content in each fraction was determined from the molecular extinction coefficient at 280 nm (Sakurai et Al., Journal of the Japanese Society for Laboratory Hematology, 16:128-135, 2015).

### <<Example 2: Preparation of monoclonal antibody>>

A monoclonal antibody was prepared using human DD/E as an immunogen in accordance with Kohler and Milstein, Nature 256: 495-7, 1975. Culture supernatants of hybridomas cloned by limiting dilution were dispensed to a 96-well microplate immobilized with an anti-mouse IgG antibody. An antibody (MIF-220) that reacted with DD/E, but did not react with fibrinogen and fibrinogen degradation products (X, Y, D₁, and E₃) was selected.

### <<Example 3: Analysis of epitope of MIF-220 antibody>>

### (1) Analysis of reactivity to fibrin/fibrinogen degradation products by ELISA

The MIF-220 antibody solution was dispersed to a 96-well microplate, and it was allowed to stand at room temperature for 1 hour or more. The microplate was washed with a 0.1 mol/L phosphate buffer (pH 7.0) containing 0.05% polyoxyethylene (20) sorbitan monolaurate (hereinafter referred to as T-PBS), and was blocked with T-PBS containing 0.3% bovine serum albumin (BSA). Various antigens were dispersed to each well, and the microplate was allowed to stand at room temperature for 1 hour. The microplate was washed with T-PBS. A peroxidase-labeled anti-human fibrinogen antibody, which had been diluted 5000 times with T-PBS containing 0.3% BSA, was added, and it was allowed to stand at room temperature for 1 hour. After washing with T-PBS, a TMB (3,3',5,5'-tetramethylbenzidine) reagent was added, and the signal was measured at 450 nm. It was judged that there was reactivity against an antigen whose absorbance exceeded 0.2. The results are shown in Table 1-A.

**[Table 1-A]**

| OD (450 nm) | | |
|---|---|---|
| Fraction | Abbreviation | ELISA signal (OD 405 nm) |
| Fibrinogen | Fbg | 0.08 |
| Fibrinogen degradation products | X | 0.06 |
| | Y | 0.05 |
| | D₁ | 0.04 |
| | E₃ | 0.04 |
| Fibrin degradation products | XDP | 2.22 |
| | DD/E | 1.85 |
| Dissociation products of DD/E | DD | 0.11 |
| | E₁₊₂ | 0.04 |
| Reconstructed product of DD and E | DD+E₁₊₂ | 0.68 |

The MIF-220 antibody reacted with XDP (polymeric XDP and DD/E), but did not react with fibrinogen and its degradation products, X, Y, D₁, and E₃. Further, it did not react with isolated DD and E₁₊₂ obtained by dissociating DD/E by urea, but reacted with a mixture of DD and E₁₊₂ (a reconstructed product).

### (2) Analysis by Western blotting

Fibrinogen, polymeric XDP, DD/E, DD, E₁₊₂, X, Y, D₁, and E₃ were subjected to SDS-Polyacrylamide gel electrophoresis (PAGE), and after transferring them to a polyvinylidene fluoride (PVDF) membrane, the reactivity of the MIF-220 antibody or an anti-human fibrinogen antibody to each antigen was measured.

The reactivity of the antibody by Western blotting of these antigens under non-reducing conditions was examined, the MIF-220 antibody did not react with any antigen. Further, also in the Western blotting performed in the absence of SDS, the MIF-220 antibody did not react with each of the aforementioned antigens.

### <<Example 4: Reactivity of MIF-220 antibody to D-E-D complex>>

### (1) Analysis by reconstruction of E-D bond (reactivity to D₁ and E)

Human D₁ and human E₁₊₂ or human E₃ suspended in TBS were mixed and reacted at 37°C for 30 minutes to reconstruct the E-D bond, and they were fractionated using a Superdex200 HR column (GE Healthcare) to compare the retention time with those of DD/E, D₁, and E₁₊₂. Further, the reactivity to the antibody was examined by ELISA. Fractions derived from other animal species were also examined in the same manner.

The results of the reactivity of the MIF-220 antibody to the D-E-D complex examined by ELISA are shown in Table 1-B.

The MIF-220 antibody did not react with each of D₁, E₁₊₂, and E₃, but reacted with the mixture of D₁ and E₁₊₂ (reconstructed product). However, the MIF-220 antibody did not react with the mixture of D₁ and E₃ (reconstructed product).

**[Table 1-B]**

| OD (450 nm) | | |
|---|---|---|
| Fraction | Abbreviation | ELISA signal (OD 405 nm) |
| Fibrinogen degradation products | D₁ | 0.00 |
| | E₃ | 0.00 |
| Fibrin degradation products | DD/E | 1.63 |
| Dissociation products of DD/E | E₁₊₂ | -0.05 |
| Reconstructed product of D and E | D₁+E₁₊₂ | 1.68 |
| | D₁+E₃ | 0.01 |

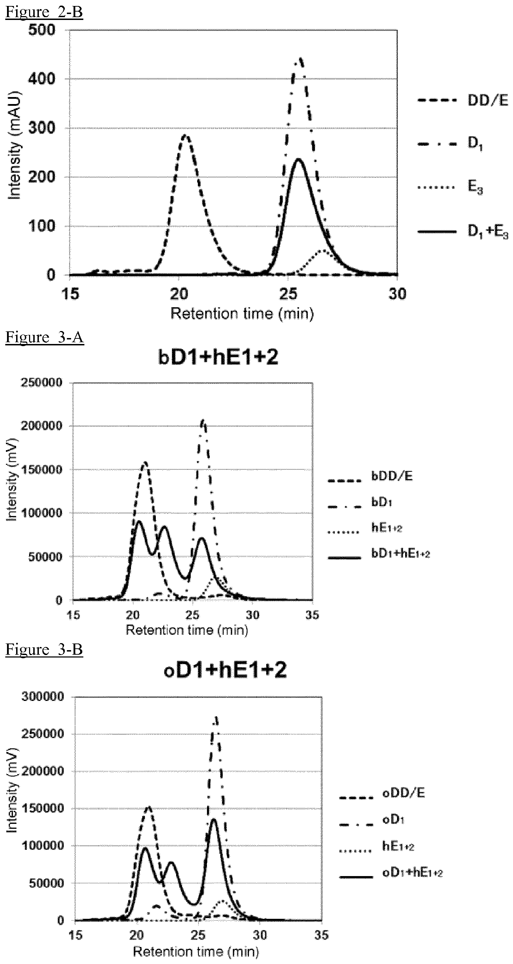

The mixture of human D₁ and human E₁₊₂ was fractionated using a Superdex200 HR column, and new peaks A and B were observed, as shown in Figure 2-A. The retention time of peak A was approximately consistent with that of DD/E, and the retention time of peak B was intermediate between DD/E and D₁. On the other hand, such a new peak was not observed in the mixture of human D₁ and human E₃, as shown in Figure 2-B.

### (2) Reactivity of human, bovine, or ovine D₁ with human E₁₊₂

The results of the reactivity of the MIF-220 antibody with DD/E of various mammals, or the reactivity of human, bovine, or ovine D₁ with human E₁₊₂ examined by ELISA are shown in Table 2.

The MIF-220 antibody reacted with human DD/E, but did not react with bovine or ovine DD/E. On the other hand, when human E₁₊₂ was mixed with human, bovine, or ovine D₁, the MIF-220 antibody reacted with any mixture. In the Table, "h" is added to human antigens, "b" is added to bovine antigens, and "o" is added to ovine antigens.

**[Table 2]**

| OD (450 nm) | | |
|---|---|---|
| | | MIF-220 |
| Human D₁ Human E₁₊₂ | hDD/E | 1.83 |
| | hD₁ | 0.09 |
| | hE₁₊₂ | 0.09 |
| | hD₁+hE₁₊₂ | 1.85 |
| Bovine D₁ + Human E₁₊₂ | bDD/E | 0.03 |
| | bD₁ | 0.03 |
| | hE₁₊₂ | 0.09 |
| | bD₁+hE₁₊₂ | 0.45 |
| Ovine D₁ + Human E₁₊₂ | oDD/E | 0.02 |
| | oD₁ | 0.03 |
| | hE₁₊₂ | 0.09 |
| | oD₁+hE₁₊₂ | 1.30 |

The results obtained by fractionating the mixture of bovine D₁ and human E₁₊₂ or the mixture of ovine D₁ and human E₁₊₂ using a Superdex200 HR column are shown in Figures 3-A and 3-B.

As in the case of the mixture of human D₁ and human E₁₊₂ (Figure 2-A), two new peaks corresponding to peaks A and B were observed, and it was confirmed that human E₁₊₂ and bovine or ovine D₁ formed the D-E-D complex, beyond the difference in animal species.

### <<Example 5: Analysis of calcium-dependency of reactivity to DD/E and D-E-D complex>>

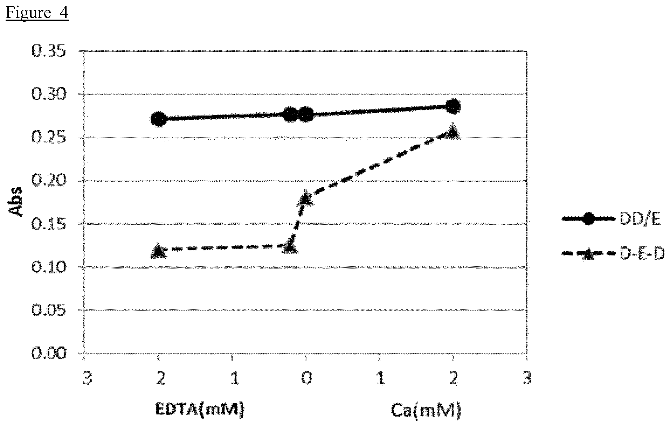

After suspending 0.3 µg/mL of human DD/E, or the human D-E-D complex obtained as peak A in Figure 2-A, in a buffer (Ca²⁺ concentration: 2 mmol/L, and EDTA concentration: 0.2 mmol/L), the DD/E or the human D-E-D complex was reacted with the immobilized MIF-220 antibody, and detected by ELISA. The results are shown in Figure 4.

The reactivity of human DD/E was not significantly affected by Ca²⁺ concentration and EDTA concentration. On the other hand, the reactivity of the D-E-D complex was almost the same as that of DD/E at a Ca²⁺ concentration of 2 mmol/L, but the reactivity of the D-E-D complex decreased significantly at a Ca²⁺ concentration of less than 2 mmol/L, and the reactivity became almost blank level at an EDTA concentration of 0.2 mmol/L or more.

From these analyses, it was shown that the MIF-220 monoclonal antibody, obtained using human DD/E as the immunogen, was an antibody that recognized a structure characteristic of XDP:(DD/E)n, because the MIF-220 antibody reacted with cross-linked fibrin degradation products XDP, but did not react with fibrinogen, and fibrinogen degradation products (X, Y, D₁, and E₃). From the fact that when DD/E (e of Figure 1-C), as the smallest unit of XDP, was dissociated into DD (f of Figure 1-C) and E₁₊₂ (g of Figure 1-C), the reactivity to each of DD and E₁₊₂ disappeared, and the fact that MIF-220 antibody did not react with each antigen of XDP and fibrinogen degradation products by Western blotting, it was shown that the MIF-220 antibody was an antibody that recognized a three-dimensional structure caused by the E-D bond.

Olexa et al. reported that when DD and E₁₊₂ were mixed, the E-D bond was again formed, and DD/E was reconstructed. In this study, it was shown that when D₁ was used instead of DD, the E-D bond could be reconstructed in the same manner (Figure 2-A). By referring to the retention time of DD/E, it was considered that peaks A and B, as shown in Figure 2-A, took the structures of D-E-D (k of Figure 1-C) and D-E (1 of Figure 1-C), respectively. On the other hand, the same authors reported that E₃ did not bind with DD, and in this study, the E-D bond was not observed as shown in Figure 2-B (As shown in h of Figure 1-C, because both A-knob and B-knob did not exist in E₃, even if E₃ was mixed with D₁, the D-E-D complex and the D-E complex were not formed, and thus, the mixture did not react with the MIF-220 antibody).

In order to determine which of the D region or the E region includes the epitope of the MIF-220 antibody, the reconstruction of the E-D bond was examined using human, bovine, and ovine DD/Es, human, bovine, and ovine D₁s, and human E₁₊₂. Since the D-E-D complex was formed in any combination of human, bovine, and ovine D₁s and human E₁₊₂ (Figure 2-A, Figure 3-A, and Figure 3-B), it was confirmed that the E-D bond is not dependent on a species difference. However, since the MIF-220 antibody reacted with the mixture of human E₁₊₂ and human, bovine, or ovine D₁, but did not react with the bovine or ovine DD/E, it was shown that the human E region was important for the reaction of the MIF-220 antibody (Table 2). From these results, it was shown that the MIF-220 antibody was one that recognized the three-dimensional structure newly generated on the human E region by the action of thrombin and the E-D bond.

The reactivity of DD/E and the D-E-D complex against the MIF-220 antibody in the presence of Ca²⁺ was nearly identical in the same amount of protein, but the reactivity against the D-E-D complex decreased drastically compared with DD/E in the presence of EDTA. As shown in e and k of Figure 1-C, the difference between DD/E and D-E-D was a cross-linked structure between D and D, and it was considered that the Ca bonding strength of the Ca binding site was stronger than the D-E-D complex due to cross-linking.

In the present invention, the chelating agent can be used in two stages. For example, when citric acid or the like is used as a chelating agent required at the time of blood collection, another chelating agent different from the chelating agent used for blood collection can be used, or the same chelating agent can be used at a different concentration, at the time of the XDP measurement. In particular, it was found that the specificity of the antibody could be raised and an accurate measurement became possible by adding a chelating agent in the reagent to reduce the influence of calcium.

DD/E is the smallest unit that constitutes XDP (d of Figure 1-B and e of Figure 1-C), and the D-E-D complex is the smallest structure that characterizes the fibrin polymer degradation products (c of Figure 1-A). It is considered that the antibody can be applied clinically for XDP detection by inhibiting the reactivity of the MIF-220 antibody to the D-E-D complex.

### INDUSTRIAL APPLICABILITY

The measurement reagent and the measurement method of the present invention can be used, for example, for measuring plasmin degradation products of cross-linked fibrin (XDP), which is useful as a diagnostic marker for disseminated intravascular coagulation (DIC).

Although the present invention has been described with reference to specific embodiments, various changes and modifications obvious to those skilled in the art are possible without departing from the scope of the appended claims.

## Claims

1. A measurement reagent for plasmin degradation products of cross-linked fibrin (XDP), said measurement reagent comprising:
(1) an anti-XDP antibody that reacts with XDP, but does not react with fibrinogen, and fragment X, fragment Y, fragment D₁, and fragment E₃, which are plasmin degradation products of fibrinogen, and
(2) a calcium chelating agent.

2. A measurement method for plasmin degradation products of cross-linked fibrin (XDP), comprising carrying out a reaction of XDP with an anti-XDP antibody in the presence of a calcium chelating agent.
